(19) Europäisches Patentamt
European Patent Office
Office européen des brevets

(11) Publication number: 0 309 953 A1

(12) # EUROPEAN PATENT APPLICATION

(21) Application number: 88115779.6

(51) Int. Cl.⁴: C07D 413/06

(22) Date of filing: 26.09.88

Claims for the following Contracting State: ES.

(30) Priority: 02.10.87 US 104627
02.10.87 US 104728
02.10.87 US 104626

(43) Date of publication of application:
05.04.89 Bulletin 89/14

(84) Designated Contracting States:
AT BE CH DE ES FR GB IT LI LU NL SE

(71) Applicant: PENNWALT CORPORATION
Pennwalt Building Three Parkway
Philadelphia-Pennsylvania 19102(US)

(72) Inventor: Georgiev, Vassil Stefanov
P.O.Box 1032
Penfield New York 14526(US)
Inventor: Mullen, George Byron
1385 Jenks Road
Avon New York 14414(US)

(74) Representative: Kraus, Walter, Dr. et al
Patentanwälte Kraus, Weisert & Partner
Thomas-Wimmer-Ring 15
D-8000 München 22(DE)

(54) **5-Substituted-3-phenyl-3-(1H-imidazol-1-ylmethyl)-2-methyl-isoxazolidines.**

(57) 5-Substituted-3-phenyl-3-(1H-imidazol-1-ylmethyl)-2-methylisoxazolidine compounds are disclosed which are useful as antifungal agents.

EP 0 309 953 A1

# 5-Substituted-3-phenyl-3-(1H-imidazol-1-ylmethyl)-2-methylisoxazolidines

## Background of the Invention

This invention pertains generally to substituted 2-methylisoxazolidines and more specifically to 5-substituted-3-phenyl-3-(1H-imidazol-1-ylmethyl)-2-methylisoxazolidines which are useful as antifungal agents.

## Brief Summary of the Invention

In accordance with this invention there are provided compounds of the formula:

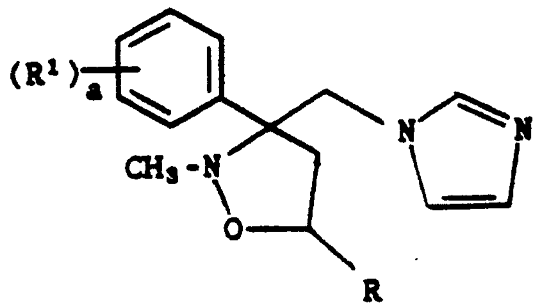

wherein;
a = 1 or 2,
$R^1$ is selected from hydrogen, lower alkyl, lower alkoxy, halogen, and combinations thereof, provided that the ortho position is hydrogen, and R is selected from:

(a) $-CH_2-O-R^2$

where $R^2$ is selected from 1-naphthyl, 2-naphthyl, and 2-oxo-1,3-benzoxathiol-6-yl;

(b) $-\underset{X}{\underset{\|}{C}}-R^2$

where X is oxygen or hydroxyimino and $R^2$ is selected from lower alkyl, lower alkoxy, substituted phenoxy and substituted phenylamino groups, wherein the phenyl substituents are selected from lower alkyl, halogen and lower alkoxy groups; and

(c)

$$-CH_2-\overset{(O)n}{S}-R^2$$

wherein n = 0 to 2 and $R^2$ is selected from lower alkyl, benzyl and (substituted phenyl)methyl groups, wherein the substituents on the phenyl rings are selected from one or more halogen, lower alkyl and lower alkoxy groups and combinations thereof;
and pharmaceutically acceptable acid addition salts thereof, in the form of their enantiomers, or mixtures of their enantiomers including diastereoisomeric pairs of such enantiomers.

## Detailed Description of the Invention

The compounds of this invention are useful as antifungal agents. They have been shown to have in vitro activity against yeast and systemic mycoses and dermatophytes as determined by broth and agar testing techniques [McGinnis, M.R., Laboratory Handbook of Medical Mycology, Academic Press, N.Y., N.Y. (1980)-]. The compounds have good to moderate inhibitory activity against a broad spectrum of organisms including Trichophyton mentagrophytes, Trichophyton tonsurans, Trichophyton rubrum, Aspergillus fumigatus, Epidermophyton floccosum, Microsporum canis, Candida albicans and Candida stellatoidea (minimum inhibitory concentration, MIC, of < .2 to 70 ug/ml).

Because of the antifungal activity of the compounds of the invention they can be used, for example, in suitable liquid, semi-solid or solid carriers in the form of solutions, emulsions, suspensions, dispersions, ointments, aerosols, soaps, detergents, and powders in amounts effective to combat systemic and dermatophylic fungal infections in warm blooded animals (1 to 20 percent active ingredient).

In the definition of the compounds of the invention, by halogen is meant chlorine, bromine, fluorine and iodine with chlorine and fluorine being preferred. By lower alkyl is meant $C_1$ to $C_4$ and by lower alkoxy is meant $C_1$ to $C_6$ which in either case can be a branched or unbranched chain.

The 5-substituted-3-phenyl-3-(1H-imidazol-1-yl-methyl)-2-methylisoxazolidines are obtained as mixtures of cis- and trans-diastereomers due to the presence in the isoxazolidine ring of two asymmetric carbon atoms. The diastereomeric mixture is conveniently separated by flash chromatography on silica gel using halogenated hydrocarbons (preferably dichloromethane and chlorofrom), alkanols (preferably methanol and ethanol), ethyl acetate and such as eluents. The eluents may be utilized alone or in combinations such as the ones comprised of 95-99% by volume halogenated hydrocarbon and 1-5% by volume alkanol. The stereochemistry of the two asymmetric carbon atoms in the isoxazolidine ring may be determined by convential methods that include X-ray crystallography, nuclear magnetic resonance spectroscopy, circular dichroism or optical dispersion. Both the cis-and trans-diastereoisomers are resolvable into their optical enantiomers with (+)- and (-)-optical rotations by standard techniques such as fractional recrystallization of the diastereomeric salts with optically active organic acids such as (+)- and (-)-tartaric acid, (+)- and (-)-dibenzoyltartaric acid and the like.

As illustrated in the following diagram, the compounds of this invention can be prepared by an initial bromination of an appropriate acetophenone and reacting the resulting bromo derivative with imidazole to produce the 1-phenyl-2-(1H-imidazol-1-yl)ethanone. Reaction of the latter with N-methylhydroxylamine hydrochloride provides the 1-phenyl-2-(1H-imidazol-1-yl)-N-methylethanimine N-oxide (1).

In one synthetic scheme the nitrone compound 1 is then treated with an appropriate alkyl aryl ether derivative 2 to give a diastereomeric mixture of the desired cis- and trans-5-{[naphthyl(or 2-oxo-1,3-benzoxathiol-6-yl)oxy] methyl}-3-phenyl-3-(1H-imidazol-1-ylmethyl)-2-methylisoxazolidines 3 (where $R^2$ is as defined in (a) above).

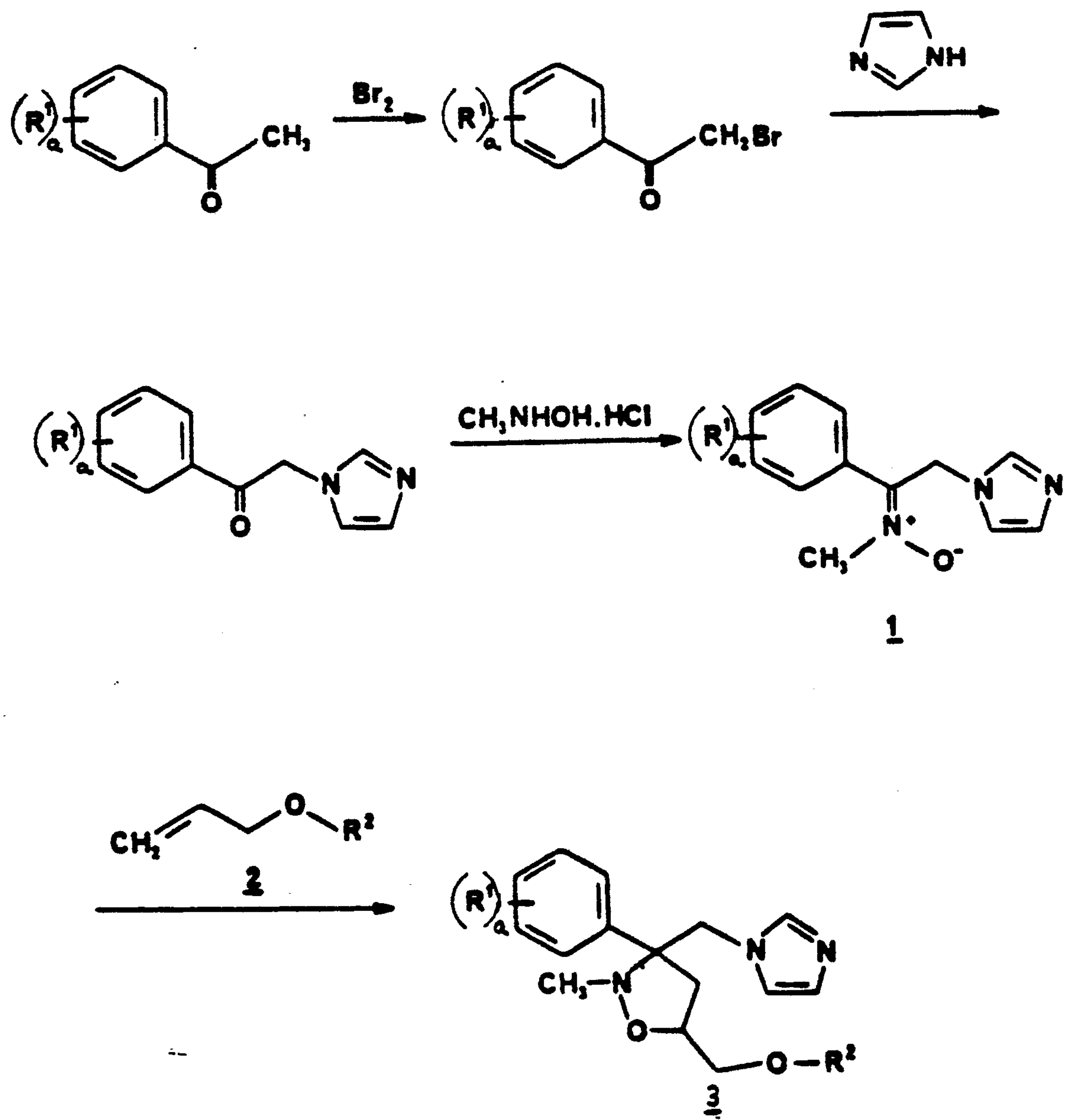


1

2

3

Similarly other compounds of the invention are prepared by reaction of the nitrone precursors 1 with:

(a) 1-alkene derivatives 4 to give a diastereomeric mixture of the desired cis- and trans-5-carbonyl derivatives of the 3-phenyl-3-(1H-imidazol-1-ylmethyl)-2-methylisoxazolidines 5 (where $R^2$ is as defined in (b) above).

Nitrone 1

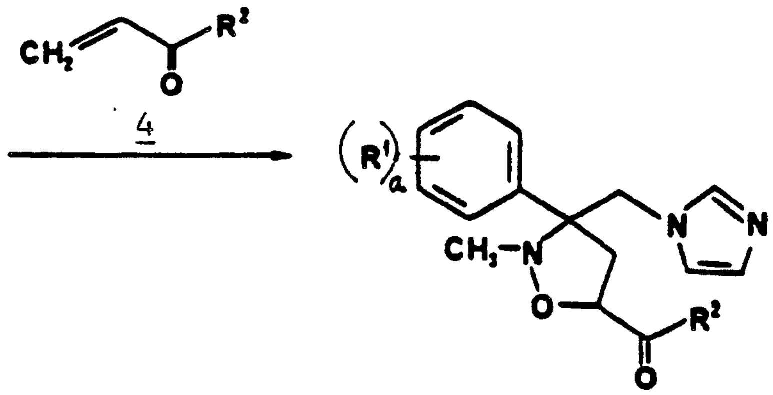

4

5

Further reaction of the ketone compounds such as 5′ with hydroxylamine hydrochloride and sodium hydroxide provides the oximes 6.

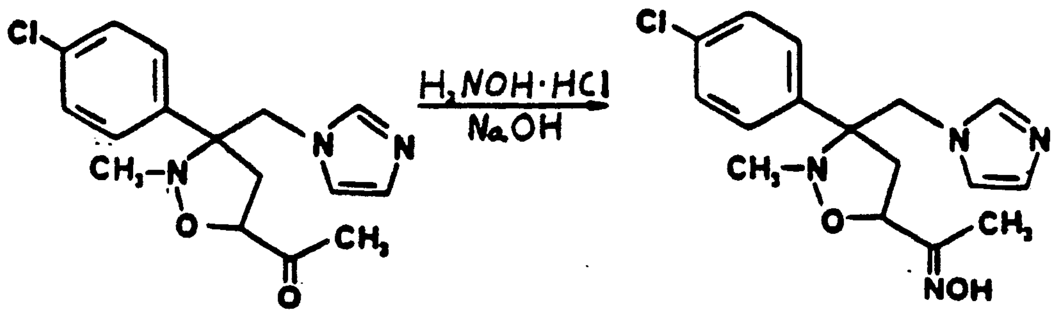


5′ 6

(b) allyl sulfide derivatives 7 to provide a diastereomeric mixture of the desired cis- and trans-5-(substituted thiomethyl)-3-phenyl-3-(1H-imidazol-1-ylmethyl)-2-methylisoxazolidine 8 (where $R^2$ is as defined in (c) above).

Nitrone 1

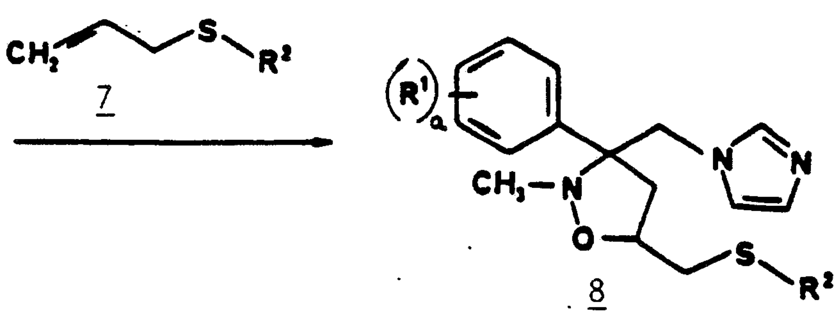

7

8

The sulfoxide derivatives are prepared by dissolving the thio derivatives in methylene chloride at low temperatures (dry ice-acetone bath) of about -80°C, adding 85% m-chloroperbenzoic acid, allowing the solution to gradually warm to room temperature, washing the resulting solution with sodium bicarbonate, drying the solution over anhydrous magnesium sulfate and then evaporating the methylene chloride to provide the crude product which is flash-chromatographed to provide pure diasteriomers.

The sulfones are prepared by dissolving the thio derivatives in methylene chloride at ambient temperature, adding 2.2 equiv. of 85% m-chloroperbenzoic acid, washing the resulting solution with sodium bicarbonate, drying the solution over anhydrous magnesium sulfate and then evapor ating the methylene chloride to provide the crude product which is flash chromatographed to provide pure diasteriomers.

The compounds of this invention are basic and thus can form salts with pharmaceutica]]y acceptable inorganic and organic acids such as, for example, acetic acid, maleic acid, malic acid, fumaric acid, succinic acid, succinamic acid, tartaric acid, citric acid, lactic acid, hydrochloric acid, hydrobromic acid, hydroiodic acid, nitric acid, sulfuric acid and phosphoric acid.

The preparation of the compounds of this invention is further illustrated by the following synthesis of intermediates and in the Examples.

## Preparation of Allyl Aryl Ethers 2

The allyl aryl ethers 2 can be prepared by the method of S. Marcinkiewicz, J., et al. Tetrahedron, 14, 208-22 (1961).

The following allyl aryl ethers were synthesized:

6-(2-Propenoxy)-1,3-benzoxathiol-2-one (2: $R^2$ = 2-oxo-1,3-benzoxathiol-6-yl); yield: 61.5%; m.p. 78-80° C-(ethyl ether).
2-(2-Propenoxy)naphthalene (2: $R^2$ = 2-naphthyl); yield: 76%; b.p. 75-80° C (0.05 mm).
1-(2-Propenoxy)naphthalene (2: $R^2$ = 1-naphthyl); yield: 74%; b.p. 75-80° C (0.05 mm).

## Example 1

3-(4-Fluorophenyl)-3-(1H-imidazol-1-ylmethyl)-2-methyl-5-{[(2-oxo-1,3-benzoxathiol-6-yl)oxy]-methyl}isoxazolidine (3, $R^1$ = 4-F, $R^2$ = 2-oxo-1,3-benzoxathiol-6-yl)

A solution of 7.00 g (0.030 mol) of 1-(4-fluorophenyl)-2-(1H-imidazol-1-yl)-N-methylethanimine N-oxide (1, $R^1$ = 4-F) [prepared by reacting 2-(1H-imidazol-1-yl)-4′-fluoroacetophenone (17.24 g, 0.0844 mol), N-methylhydroxylamine hydrochloride (10.29 g, 0.123 mol) and sodium acetate (10.16 g, 0.124 mol) in 250 ml ethanol] and 6.56 g (0.0315 mol) of 6-(2-propenoxy)-1,3-benzoxathiol-2-one (2, $R^2$ = 2-oxo-1,3-benzoxathiol-6-yl) in 200 ml of toluene is heated to reflux under a nitrogen atmosphere and stirred for 48 hours, then cooled to ambient temperature and extracted with water (2 x 100 ml). The organic phase is dried over anhydrous magnesium sulfate and concentrated in vacuo to a dark oil containing a cis- and trans-diastereomeric mixture of compound 3 ($R^1$ = 4-F, $R^2$ = 2-oxo-1,3-benzoxathiol-6-yl), which is flash-chromatographed on neutral silica gel using ethyl acetate as the eluent.

Isomer A (4.75 g, 36%) has a melting point of 140-142° C (ethyl acetate). Anal. Calcd. for $C_{22}H_{20}FN_3O_4S$: C, 59.85; H, 4.57; F, 4.30; N, 9.52; S, 7.26. Found: C, 60.03; H, 4.59; F, 4.33; N, 9.48; S, 7.40.

## Example 2

3-(4-Fluorophenyl)-3-(1H-imidazol-1-ylmethl)-2-methyl-5-{[(2-naphthyl)oxy]methyl}isoxazolidine (3, $R^1$ = 4-F, $R^2$ = 2-naphthyl)

Compound 3 ($R^1$ = 4-F, $R^2$ = 2-naphthyl) is prepared by a method similar to that described in Example 1 from 8.17 (0.035 mol) of 1-(4-fluorophenyl)-2-(1H-imidazol-1-yl)-N-ethanimine N-oxide (1, R = 4-F) and 8.30 g (0.045 mol) of 2-(2-propenoxy)naphthalene (2, $R^2$ = 2-naphthyl) in 200 ml of toluene. The resulting cis- and trans-diastereomeric mixture of compound 3 ($R^1$ = 4-F, $R^2$ = 2-naphthyl) is flash-chromatographed on neutral silica gel using a 98:2 by volume mixture of chloroform and methanol as the eluent.

Isomer A (3.01 g, 20.6%) has a melting point of 160-162° C (ethyl acetate). Anal. Calcd. for $C_{25}H_{24}FN_3O_2$ - (adjusted for 0.59% $H_2O$): C, 71.50; H, 5.82; F, 4.52; N, 10.01. Found: C, 71.33; H, 5.72; F, 4.50; N, 10.05.

## Example 3

3-(1H-Imidazol-1-ylmethyl)-2-methyl-5-{[(1-naphthyl)oxy]methyl}-3-phenylisoxazolidine (3, $R^1$ = H, $R^2$ = 1-naphthyl)

Compound 3 ($R^1$ = H, $R^2$ = 1-naphthyl) is prepared by a method similar to that described in Example 1 from 2-(1H-imidazol-l- yl)-N-methyl-1-phenylethanimine N-oxide (1, $R^1$ = H) (10.28 g, 0.0477 mol) and 1-(2-propenoxy)naphthalene (2, $R^2$ = 1-naphthyl) (10.90 g, 0.0592 mol) in 200 ml of toluene. The resulting cis- and trans-diastereomeric mixture of compound 3 ($R^1$ = H, $R^2$ - 1-naphthyl) is flash-chromatographed on neutral silica gel using a 98:2 mixture of chloroform and methanol as the eluent.
Isomer A (7.84 g, 41%) has a melting point of 42-45° C (ethyl acetate). Isomer A as the mononitrate salt has a melting point of 160-163° C (decomp.). Anal. Calcd. for $C_{25}H_{25}N_3O_2$ • $HNO_3$: C, 64.92; H, 5.67; N, 12.11. Found: C, 64.85; H, 5.73; N, 12.15

## Example 4

3-(1H-Imidazol-1-ylmethyl)-2-methyl-5-[(naphthyloxy)methyl]-3-(substituted phenyl)isoxazolidines

By following the method of Examples 2 and 3 and substituting for 1-(4-fluorophenyl)-2-(1H-imidazol-1-yl)-N-methylethanimine N-oxide one of the following compounds:
1-(4-methoxyphenyl)-2-(1H-imidazol-1-yl)-N-methylethanimine N-oxide,
1-(4-methylphenyl)-2-(1H-imidazol-1-yl)-N-methylethanimine N-oxide,
1-(4-chloro-3-methylphenyl)-2-(1H-imidazol-1-yl)-N-methyl ethanimine N-oxide,
1-(3,4-dichlorophenyl)-2-(1H-imidazol-1-yl)-N-methylethanimine oxide,
1-(3-methylphenyl)-2-(1H-imidazol-1-yl)-N-methylethanimine N-oxide, or
1-(3-methoxyphenyl)-2-(1H-imidazol-1-yl)-N-methylethanimine N-oxide,
the corresponding 3-(1H-imidazol-1-ylmethyl)-2-methyl-5-[(naphthyloxy)methyl]-3-(substitued phenyl)-isoxazolidines can be prepared.

## Preparation of Acrylic Acid Derivatives 4

4-Methylphenyl acrylate, 4-chlorophenyl acrylate and N-(4-chlorophenyl)acrylamide can be prepared from commercially available acryloyl chloride by using standard literature procedures. For example, reaction of 4-chloroaniline with 1 equivalent of acryloyl chloride in tetrahydrofuran solution containing 1 equivalent of triethylamine gave N-(4-chlorophenyl)acrylamide.

## Example 5

Ethyl 3-(4-Chlorophenyl)-3-(1H-imidazol-1-ylmethyl)-2methylisoxazolidine-5-carboxylate (5, $R^1$ = 4-Cl, $R^2$ = OEt)

A solution of 22.47 g (0.090 mol) of 1-(4-chlorophenyl)-2-(lH -imidazol-1-yl)-N-methylethanimine N-oxide (1, $R^1$ = 4-Cl) [which can be prepared by reacting 2-(1H-imidazol-1-yl)-4′- chloroacetophenone (38.77 g, 0.176 mol), N-methylhydroxylamine hydrochloride (17.63 g, 0.211 mol) and sodium acetate (34.6 g, 0.422 mol) in 470 ml ethanol] and 20 ml (0.18 mol) of ethyl acrylate (4, $R^2$ = OEt) in 150 ml of toluene is refluxed for 45 hours under a nitrogen atmosphere, cooled to ambient temperature and concentrated in vacuo. The residual dark oil, containing a cis- and trans-diastereomeric mixture of compound 5 ($R^1$ = 4-Cl, $R^2$ = OEt),

is flash-chromatographed on neutral silica gel using ethyl acetate as eluent.

Isomer A (1.94 g, 6.2%) has a melting point of 150-152 °C (ethyl acetate). Anal. Calcd. for $C_{17}H_{20}ClN_3O_3$: C, 58.37; H, 5.76; Cl, 10.13; N, 12.01. Found: C, 58.39; H, 5.93; Cl, 10.11; N, 12.03.

## Example 6

4-Methylphenyl 3-(1H-Imidazol-1-ylmethyl)-2-methyl-3-phenylisoxazolidine-5-carboxylate (5, $R^1$ = H, $R^2$ = $OC_6H_4CH_3$-4)

A solution of 7.60 g (0.043 mol) of 2-(1H-imidazol-1-yl) -N-methyl-1-phenylethanimine N-oxide (1, $R^1$ = H) and 10.55 g (0.065 mol) of 4-methylphenyl acrylate (4, $R^2$ = $OC_6H_4CH_3$-4) in 200 ml of toluene is heated to 55-60 °C and stirred for 18 hours under a nitrogen atmosphere, then cooled to ambient temperature and concentrated in vacuo, leaving a dark viscous oil containing a cis- and trans-diastereomeric mixture of compound 5 ($R^1$ = H, $R^2$ = $OC_6H_4CH_3$-4). Isomer A is obtained by crystallization from ethyl ether; yield: 3.80 g (23%).

Isomer A has a melting point of 149-151 °C (ethyl acetate). Anal. Calcd. for $C_{22}H_{23}N_3O_3$: C, 70.01; H, 6.14; N, 11.13. Found: C, 69.98; H, 6.24; N, 11.17.

## Example 7

4-Chlorophenyl 3-(4-Fluorophenyl)-3-(1H-imidazol-1-ylmethyl) -2-methylisoxazolidine-5-carboxylate (5, $R^1$ = 4-F, $R^2$ = $OC_6H_4Cl$-4)

Compound 3 ($R^1$ = 4-F, $R^2$ = $OC_6H_4Cl$-4) is prepared by a method similar to that described in Example 6 from 1-(4-fluorophenyl)-2-(1H-imidazol-1-yl)-N-methylethanimine N-oxide (1, $R^1$ = 4-F) and 4-chlorophenyl acrylate (4, $R^2$ = $OC_6H_4Cl$-4). The resulting cis- and trans-diastereomeric mixture of compound 5 ($R^1$ = 4-F, $R^2$ = $OC_6H_4Cl$-4) is flash-chromatographed on neutral silica gel using ethyl acetate as eluent. Isomer A has a melting point of 146-148 °C (ethyl ether).

## Example 8

4-Chlorophenyl 3-(4-Chlorophenyl)-3-(1H-imidazol-1-ylmethyl) -2-methylisoxazolidine-5-carboxylate (5, $R^1$ = 4-Cl, $R^2$ = $OC_6H_4Cl$-4)

Compound 5 ($R^1$ = 4-Cl, $R^2$ = $OC_6H_4Cl$-4) is prepared by a method similar to that described in Example 6 from 1-(4-chlorophenyl)-2-(1H-imidazol-1-yl)-N-methylethanimine N-oxide (1, $R^1$ = 4-Cl) and 4-chlorophenyl acrylate (4, $R^2$ = $OC_6H_4Cl$-4). The resulting cis- and trans-diastereomeric mixture of compound 5 ($R^1$ = 4-Cl, $R^2$ = $OC_6H_4Cl$-4) is flash-chromatographed on neutral silica gel using ethyl acetate as eluent.

Isomer A has a melting point of 141-144 °C (ethyl acetate).

## Example 9

N-(4-Chlorophenyl) 3-(4-Chlorophenyl)-3-(1H-imidazol-1-ylmethyl)-2-methylisoxazolidine-5-carboxamide (5, $R^1$ = 4-Cl, $R^2$ = $NHC_6H_4Cl$-4)

A solution of 34.1 g (0.14 mol) of 1-(4-chlorophenyl)-2-(1H-imidazol-1-yl)-N-methylethanimine N-oxide (1, $R^1$ = 4-Cl) and 34.4 g (0.19 mol) of N-(4-chlorophenyl)acrylamidc (4, $R^2$ = $NHC_6H_4Cl$-4) in 300 ml of toluene is refluxed for 18 hours under a nitrogen atmosphere, cooled to ambient temperature and concentrated in vacuo. The residual dark oil, containing a cis- and trans-diastereomeric mixture of compound 5 ($R^1$ = 4-Cl, $R^2$ = $NHC_6H_4Cl$-4), is flash-chromatographed on neutral silica gel using a 99:1 by volume mixture of chloroform and methanol as eluent.

Isomer A (12.73 g, 21%) has a melting point of 197-198°C (ethyl acetate). Anal. Calcd. for $C_{21}H_{20}Cl_2N_4O_2$: C, 58.48; H, 4.67; Cl, 16.44; N, 12.99. Found: C, 58.67; H, 4.82; Cl, 16.21; N, 13.01.

Isomer B (5.72 g, 9.5%) has a melting point of 204-205°C (ethyl acetate). Anal. Calcd. for $C_{21}H_{20}Cl_2N_4O_2$: C, 58.48; H, 4.67; Cl, 16.44; N, 12.99. Found: C, 58.53; H, 4.76; Cl, 16.34; N, 13.03.

## Example 10

1-[3-(4-Chlorophenyl)-3-(1H-imidazol-1-ylmethyl)-2-methylisoxazolidin-5-yl]ethanone (5, $R^1$ = 4-Cl, $R^2$ = $CH_3$)

A solution of 16.20 g (0.0649 mol) of 1-(4-chlorophenyl)-2-(1H-imidazol-1-yl)-N-methylethanimine N-oxide (1, $R^1$ = 4-Cl) and 6.42 ml (0.0791 mol) of methyl vinyl ketone (4, $R^2$ = $CH_3$) in 450 ml of toluene is heated to reflux for 4 hours under a nitrogen atmosphere, cooled to ambient temperature and concentrated in vacuo. The residual dark oil, containing a cis- and trans-diastereomeric mixture of compound 5 ($R^1$ = 4-Cl, $R^2$ = $CH_3$) is flash-chromatographed on neutral silica gel using a 98:2 by volume mixture of chloroform and methanol as eluent.

Isomer A (12.27 g, 59%) has a melting point of 147-149°C (ethyl acetate). Anal. Calcd. for $C_{16}H_{18}ClN_3O_2$: C, 60.09; H, 5.67; Cl, 11.09; N, 13.14. Found: C, 60.14; H, 5.85; Cl, 11.26; N, 13.17.

## Example 11

1-[3(4-Chlorophenyl)-3-(1H-imidazol-1-ylmethyl)-2-methylisoxazolidin-5-yl]ethanone oxime (6)

Under a nitrogen atmosphere, a solution of 3.00 g (0.00938 mol) of 1-[3-(4-chlorophenyl)-3-(1H-imidazol-1-ylmethyl)-2-methylisoxazolidin-5-yl]ethanone [isomer A (5, $R^1$ = 4-Cl, $R^2$ = $CH_3$)], 0.69 g (0.010 mol) of hydroxylamine hydrochloride and 0.60 g (0.015 mol) of sodium hydroxide in 75 ml of ethanol is refluxed for 1 hour, cooled to ambient temperature, neutralized with 0.1 N hydrochloric acid, and extracted with chloroform (2 x 50 ml). The combined organic extract is washed with 50 ml of saturated aqueous sodium chloride, dried over anhydrous magnesium sulfate and concentrated in vacuo to give a white solid. Crystallization from ethanol gives 1.57 g (50%) of compound 6, having a melting point of 214-216°C (ethanol). Anal. Calcd. for $C_{16}H_{19}ClN_4O_2$: C, 57.40; H, 5.72; Cl, 10.59; N, 16.73. Found: C, 57.24; H, 5.80; Cl, 10.70; N, 16.62.

## Example 12

3-(1H-Imidazol-1-ylmethl)-2-methyl-3-(substituted phenyl) -5-carboxylic acid 4-methoxyphenyl esters

By following the method of Example 6 and substituting 4-methoxyphenyl acrylate for 4-methylphenyl acrylate, and substituting for 2-(1H-imidazol-1-yl)-N-methyl-1-phenylethanimine N-oxide one of the following compounds:
1-(4-methoxyphenyl)-2-(1H-imidazol-1-yl)-N-methylethanimine N-oxide,
1-(4-methylphenyl)-2-(1H-imidazol-1-yl)-N-methylethanimine N-oxide,
1-(4-chloro-3-methylphenyl)-2-(1H-imidazol-1-yl)-N-methylethanimine N-oxide,
1-(3,4-dichlorophenyl)-2-(1H-imidazol-1-yl)-N-methylethanimine N-oxide,
1-(3-methoxyphenyl)-2-(1H-imidazol-1-yl)-N-methylethanimine N-oxide, or
1-(3-methylphenyl)-2-(1H-imidazol-1-yl)-N-methylethanimine, the corresponding 3-(1H-imidazol-1-ylmethyl)-2-methyl-3-(substituted phenyl)-5-carboxylic acid 4-methoxyphenyl esters can be prepared.

Preparation of Allyl Sulfides 7

The allyl sulfides 7 can be prepared by reaction of the corresponding alkyl mercaptans with sodium hydride and allyl bromide in ether. Allyl sulfides 7 [$R^2$ = $CH_3$, $CH(CH_3)_2$] are commercially available.

## Example 13

3-(4-Chlorophenyl)-3-(1H-imidazol-1-ylmethyl)-2-methyl-5-[(methylthio)methyl]isoxazolidine (8, $R^1$ = 4-Cl, $R^2$ = $CH_3$)

A solution of 16.50 g (0.0661 mol) of 1-(4-chlorophenyl)-2-(1-imidazol-1-yl)-N-methylethanimine N-oxide (1, $R^1$ = 4-Cl) [prepared by reacting 2-(1H-imidazol-1-yl) -4′-chloroacetophenone (22.05 g, 0.10 mol), N-methylhydroxylamine hydrochloride (10.65 g, 0.127 mol) and sodium bicarbonate (10.84 g, 0.129 mol) in 300 ml ethanol] and 15.0 ml (0.137 mol) of allyl methyl sulfide (7, $R^2$ = $CH_3$) in 150 ml of toluene was heated to reflux under a nitrogen atmosphere and stirred for 30 hours, then cooled to ambient temperature and concentrated in vacuo. The residual dark oil, containing a cis- and trans-diastereomeric mixture of compound 8 ($R^1$ = 4-Cl, $R^2$ = $CH_3$) was flash-chromatographed on neutral silica gel using a 98:2 by volume mixture of chloroform and methanol as eluent.

Isomer A (4.53 g, 20%) has a melting point of 142-144°C (ethyl acetate). Anal. Calcd. for $C_{16}H_{20}ClN_3OS$: C, 56.88; H, 5.97; Cl, 10.49; N, 12.44; S, 9.49. Found: C, 56.95; H, 6.01; Cl, 10.21; N, 12.39; S, 9.63.

## Example 14

3-(1H-Imidazol-1-ylmethyl)-2-methyl-5-{[(1-methylethyl)thio]methyl}-3-phenylisoxazolidine [8, $R^1$ = H, $R^2$ = $CH(CH_3)_2$]

Compound 8 [$R^1$ = H, $R^2$ = $CH(CH_3)_2$] was prepared by a method similar to that described in Example 13 from 5.19 g (0.0241 mol) of 2-(1H-imidazol-1-yl)-N-methyl-1-phenylethanimine N-oxide (1, $R^1$ = H) and 2.80 g (0.0241 mol) of allyl isopropyl sulfide [7, $R^2$ = $CH(CH_3)_2$]. The resulting cis- and trans-diastereomeric mixture of compound 8 [$R^1$ = H, $R^2$ = $CH(CH_3)_2$] was flash-chromatographed on neutral silica gel using a 99:1 by volume mixture of chloroform and methanol as eluent.

Isomer A (2.48 g, 31%) has a melting point of 57.5-59°C (ether:cyclohexane, 1:2). Anal. Calcd. for $C_{18}H_{25}N_3OS \cdot HCl$: C, 58.76; H, 7.12; Cl, 9.64; N, 11.42; S, 8.71. Found: C, 58.48; H, 7.12; Cl, 9.88; N, 11.38; S, 8.90.

## Example 15

5-{[[(3,4-Dichlorophenyl)methyl]thio]methyl}-3-(1H-imidazol-1-ylmethyl-2-methyl-3-phenylisoxazolidine (8; $R^1$ = H, $R^2$ = $CH_2C_6H_3Cl_2$-3,4)

Compound 8 ($R^1$ = H, $R^2$ = $CH_2C_6H_3Cl_2$-3,4) was prepared by a method similar to that described in Example 13 from 5.00 g (0.023 mol) of 2-(1H-imidazol-1-yl)-N-methyl-1-phenylethanimine N-oxide (1, $R^1$ = H) and 8.04 g (0.0345 mol) of allyl (3,4-dichlorophenyl)methyl sulfide (7, $R^2$=$CH_2C_6H_3Cl_2$-3,4). The resulting cis- and trans-diastereomeric mixture of compound 8 ($R^1$ = H, $R^2$ = $CH_2C_6H_3Cl_2$-3,4) was flash-chromatographed on neutral silica gel using a 99:1 by volume mixture of chloroform and methanol as eluent. Isomer A (0.93 g, 9%) has a melting point of 105.5-108 °C (ethyl acetate). Anal. Calcd. for $C_{22}H_{23}Cl_2N_3OS$: C, 58.93; H, 5.17; Cl, 15.81; N, 9.37; S, 7.15. Found: C, 58.66; H, 5.00; Cl, 15.96; N, 9.26; S, 7.23.

## Example 16

5-{[[(4-Chlorophenyl)methyl]thio]methyl}-3-(1H-imidazol-1-ylmethyl)-2-methyl-3-phenylisoxazolidine (8, $R^1$ = H, $R^2$ = $CH_2C_6H_4Cl$-4)

Compound 8 ($R^1$ = H, $R^2$ = $CH_2C_6H_4Cl$-4) was prepared by a method similar to that described in Example 13 from 4.60 g (0.021 mol) of 2-(1H-imidazol-1-yl)-N-methyl-1-phenyl-ethanimine N-oxide (1, $R^1$ = H) and 5.0 g (0.023 mol) of allyl (4-chlorophenyl)methyl sulfide (7, $R^2$ = $CH_2C_6H_4Cl$-4). The resulting cis- and trans-diastereomeric mixture of compound 8 ($R^1$ = H, $R^2$ = $CH_2C_6H_4Cl$-4) was flash-chromatographed on neutral silica gel using a 99:1 by volume mixture of chloroform and methanol as eluent.

Isomer A•HCl (1.31 g, 13.6%) has a melting point of 181-185 °C (ether). Anal. Calcd. for $C_{22}H_{25}Cl_2N_3OS$: C, 58.66; H, 5.59; Cl, 15.74; N, 9.33; S, 7.12. Found: C, 58.10; H, 5.62; Cl, 15.72; N, 9.20; S, 7.38.

## Example 17

5-[[[(Substituted phenyl)methyl]thio]methyl]-3-(1H-imidazol-1-ylmethyl)-2-methyl-3-(substituted phenyl)-isoxazolidines

By following essentially the same method as Example 16 and substituting allyl (4-methylphenyl)methyl sulfide or allyl (4-methoxyphenyl)methyl sulfide for allyl (4-chlorophenyl)methyl sulfide and substituting for 2-(1H-imidazol-1-yl)-N-methyl-1-phenylethanimine N-oxide one of the following compounds:
1-(4-methoxyphenyl)-2-(1H-imidazol-1-yl)-N-methylethanimine N-oxide,
1-(4-methylphenyl)-2-(1H-imidazol-1-yl)-N-methylethanimine N-oxide,
1-(4-chloro-3-methylphenyl)-2-(1H-imidazol-1-yl)-N-methylethanimine N-oxide,
1-(3,4-dichlorophenyl)-2-(1H-imidazol-1-yl)-N-methylethanimine oxide,
1-(3-methylphenyl)-2-(1H-imidazol-1-yl)-N-methylethanimine N-oxide, or
1-(3-methoxyphenyl)-2-(1H-imidazol-1-yl)-N-methylethanimine N-oxide,
the corresponding 5-[[[(substituted phenyl)methyl]thio]methyl]-3-(1H-imidazol-1-ylmethyl)-2-methyl-3-(substituted phenyl)isoxazolidines are prepared.

Salts of the compounds of the invention are prepared as known in the art, for example, by dissolving the compound in a 10:1 by volume mixture of ethanol and aqueous acid such as HCl (in Examples 14 and 16) or $HNO_3$, evaporating the solvent, and then recrystallizing the crude salt, for example, from methanol-ether, 1:3 by volume in the case of HCl salts, and ethanol in the case of $HNO_3$ salts.

**Claims**

1. A compound of the formula:

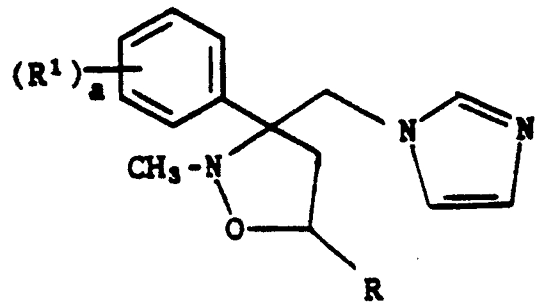

wherein;
a = 1 or 2,
$R^1$ is selected from hydrogen, lower alkyl, lower alkoxy, halogen, and combinations thereof, provided that the ortho position is hydrogen, and R is selected from:
(a) $-CH_2-O-R^2$
where $R^2$ is selected from 1-naphthyl, 2-naphthyl, and 2-oxo-1,3-benzoxathiol-6-yl;
(b) $-\underset{\overset{\|}{X}}{C}-R^2$
where X is oxygen or hydroxyimino and $R^2$ is selected from lower alkyl, lower alkoxy, substituted phenoxy and substituted phenylamino groups, wherein the phenyl substituents are selected from lower alkyl, halogen and lower alkoxy groups; and
(c)

$$-CH_2-\overset{(O)n}{S}-R^2$$

wherein n = 0 to 2 and $R^2$ is selected from lower alkyl, benzyl and (substituted phenyl)methyl groups, wherein the substituents on the phenyl rings are selected from one or more halogens, lower alkyl and lower alkoxy groups and combinations thereof;
and the pharmaceutically acceptable acid addition salts thereof, in the form of their enantiomers, or mixtures of their enantiomers including diastereoisomeric pairs of such enantiomers.

2. A compound according to Claim 1 wherein R is:
$-CH_2-O-R$
where $R^2$ is selected from 1-naphthyl, 2-naphthyl, and 2-oxo-1,3-benzoxathiol-6-yl.

3. A compound according to Claim 1 wherein R is:
$-\underset{\overset{\|}{X}}{C}-R^2$
where X is oxygen or hydroxyimino and $R^2$ is selected from lower alkyl, lower alkoxy, substituted phenoxy and substituted phenylamino groups, wherein the phenyl substituents are selected from lower alkyl, halogen and lower alkoxy groups.

4. A compound according to Claim 1 wherein R is:

$$-CH_2-\overset{(O)n}{S}-R^2$$

wherein n = 0 to 2 and $R^2$ is selected from lower alkyl, benzyl and (substituted phenyl)methyl groups, wherein the substituents on the phenyl rings are selected from one or more halogens, lower alkyl and lower alkoxy groups and combinations thereof.

5. A compound according to claim 2 wherein the compound is 3-(4-fluorophenyl)-3-(1H-imidazol-1-ylmethyl)-2-methyl-5-{[(2-oxo-1,3-benzoxathiol-6-yl)oxy]methyl}isoxazolidine.

6. A compound according to claim 2 wherein the compound is 3-(4-fluorophenyl)-3-(1H-imidazol-1-ylmethyl)-2-methyl-5-{[(2-naphthyl)oxy]methyl}isoxazolidine.

7. A compound according to claim 2 wherein the compound is 3-(1H-imidazol-1-ylmethyl)-2-methyl-5-{[(1-naphthyl)oxy]methyl}-3-phenylisoxazolidine.

8. A compound according to claim 3 wherein the compound is ethyl 3-(4-chlorophenyl)-3-(1H-imidazol-1-ylmethyl)-2-methylisoxazolidine-5-carboxylate.

9. A compound according to claim 3 wherein the compound is 4-methylphenyl 3-(1H-imidazol-1-ylmethyl)-2-methyl-3- phenylisoxazolidine-5-carboxylate.

10. A compound according to claim 3 wherein the compound is N-(4-chlorophenyl) 3-(4-chlorophenyl)-3-(1H-imidazol-1-ylmethyl)-2-methylisoxazolidine-5-carboxamide.

11. A compound according to claim 3 wherein the compound is 1-[3-(4-chlorophenyl)-3-(1H-imidazol-1-ylmethyl)-2-methylisoxazolidin-5-yl]ethanone.

12. A compound according to claim 3 wherein the compound is 1-[3-(4-chlorophenyl)-3-(1H-imidazol-1-ylmethyl)-2-methylisoxazolidin-5-yl]ethanone oxime.

13. A compound according to claim 4 wherein the compound is 3-(4-chlorophenyl)-3-(1H-imidazol-1-ylmethyl)-2-methyl-5-[(methylthio)methyl]isoxazolidine.

Claim for the following Contracting State: Spain

1. A process for preparation of compounds of the formula:

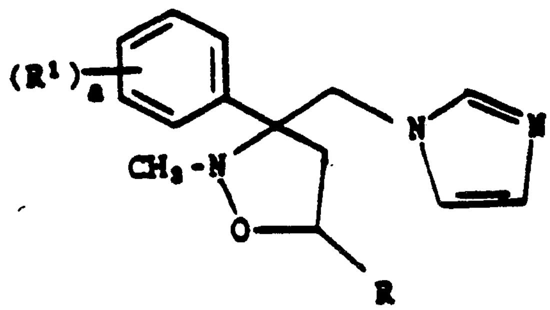

wherein;
a = 1 or 2,
$R^1$ is seleoted from hydrogen, lower alkyl, lower alkoxy, halogen, and combinations thereof. provided that the ortho position is hydrogen, and R is selected from:
(a) $-CH_2-O-R^2r$
where $R^2$ is selected from 1-naphthyl, 2.naphthyl, 2-oxo.1,3-benzoxathiol.6-yl;
(b) $-\underset{X}{\overset{\|}{C}}-R^2$
where X is oxygen or hydroxyimino and $R^2$ is selected from lower alkyl, lower alkoxy, substituted phenoxy and substituted phenylamino groups, wherein the phenyl substituents are selected from lower alkyl, halogen
(c)

$$-CH_2-\overset{(O)n}{S}-R^2$$

wherein n = 0 to 2 and $R^2$ is selected from lower alkyl, benzyl and (substituted phenyl)methyl groups, wherein the substituents on the phenyl rings are selected from one or more halogens, lower alkyl and lower alkoxy groups and combinations thereof; characterized by:
a: treating an acetophenone of the formula:

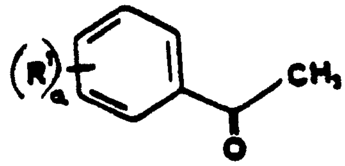

wherein $R^1$ and a are as defined hereinabove with bromine;

b: treating the brominated derivative produced in step a above with imidazole to form a compound of the formula:

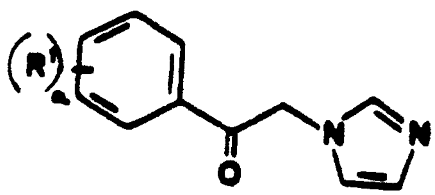

wherein $R^1$ and a are as defined hereinabove;

c: treating the 1-phenyl-2-(1H-imidazol-1-yl)ethanone produced in step b above with methyl hydroxylamine hydrochloride to produce a nitrone of the formula:

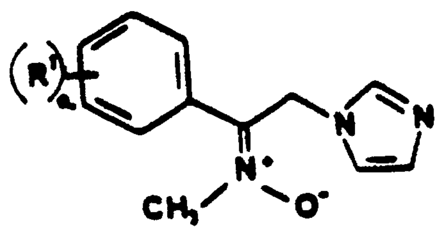

wherein $R^1$ and a are as defined hereinabove;

d: treating the nitrone produced in step c above with a compound selected from the group consisting of compounds of the formula

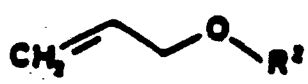

,

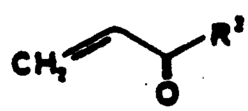

and

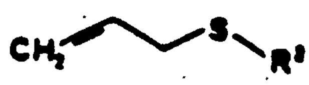

wherein $R^2$ is as defined hereinabove; and

e: optionally treating a ketocompound produced in step d with hydroxylamine hydrochloride, treating a sulfide derivative produced in step d with m-chloroperbenzoic acid at about -85°C to produce a sulfoxide, or treating the sulfide produced in step d with m-chloroperbenzoic acid at about ambient temperature to produce a sulfone.

2. A process according to Claim 1 wherein R is:

$-CH_2-O-R^2$

where $R^2$ is selected from 1-naphthyl, 2-naphthyl, and 2-oxo-1,3-benzoxathiol-6-yl.

3. A process according to Claim 1 wherein R is:

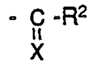

where X is oxygen or hyrdoxyimino and $R^2$ is selected from lower alkyl, lower alkoxy, substituted phenoxy and substituted phenylamino groups, wherein the phenyl substituents are selected from lower alkyl, halogen and lower alkoxy groups.

4. A process according to Claim 1 wherein R is:

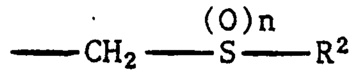

wherein n = 0 to 2 and $R^2$ is selected from lower alkyl, benzyl and (substituted phenyl)methyl groups, wherein the substituents on the phenyl rings are selected from one or more halogens, lower alkyl and lower alkoxy groups and combinations thereof.

5. A process according to Claim 2 wherein $R^1$ is 4-fluoro and $R^2$ is 2-oxo-1,3-benzoxathiol-6-yl.

6. A process according to Claim 2 wherein $R^1$ is 4-fluoro and $R^2$ is 2-naphthyl.

7. A process according to Claim 2 wherein $R^1$ is hydrogen and $R^2$ is 1-naphthyl.

8. A process according to Claim 3 wherein $R^1$ is 4-chloro and $R^2$ is ethoxy.

9. A process according to Claim 3 wherein $R^1$ is hydrogen and $R^2$ is 4-methylphenoxy.

10. A process according to Claim 3 wherein $R^1$ is 4-chloro and $R^2$ is $NC_6H_4Cl$-4,

11. A process according to Claim 3 wherein $R^1$ is 4-chloro and $R^2$ is methyl.

12. A process as defined in Claim 1 wherein 1-[3-(4-chlorophenyl)-3-(1H-imidazol-1-ylmethyl)-2-methylisoxazolidine-5-yl]ethanone is treated with hydroxylamine hydrochloride to give 1-[3-(4-chlorophenyl)-3-(1H-imidazol-1-ylmethyl)-2-methyl-isoxazolidine-5-yl] ethanone oxime.

13. A process as defined in Claim 4 wherein $R^1$ is 4-chloro and $R^2$ is methyl.

14. Use of compounds produced by any of Claims 1 through 13 as antifungal agents.

European Patent Office

# EUROPEAN SEARCH REPORT

Application number

EP 88115779.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,A | EP - A2 - 0 257 391 (PENNWALT) * Claim 1 * -- | 1 | C 07 D 413/06 |
| P,A | US - A - 4 719 306 (GEORGIEV) * Claim 1 * -- | 1 | |
| P,A | US - A - 4 723 021 (GEORGIEV) * Claim 1 * -- | 1 | |
| P,A | US - A - 4 727 156 (GEORGIEV) * Claim * ---- | 1 | |

TECHNICAL FIELDS SEARCHED (Int. Cl.4)

C 07 D 413/00

The present search report has been drawn up for all claims

| Place of search | Date of completion of the search | Examiner |
|---|---|---|
| VIENNA | 28-11-1988 | HAMMER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document